# EUROPÄISCHE PATENTSCHRIFT

(11) **EP 4 547 132 B1**
(45) Veröffentlichungstag und Bekanntmachung des Hinweises auf die Patenterteilung: **14.10.2026**
(21) Anmeldenummer: 23737935.9
(22) Anmeldetag: 29.06.2023
(51) Int. Cl.: A61B 17/29, A61B 17/00

(54) **VERBINDUNGSVORRICHTUNG FÜR ZWEI SCHAFTBAUTEILE, CHIRURGISCHES INSTRUMENT MIT EINER SOLCHEN VERBINDUNGSVORRICHTUNG UND VERFAHREN ZUM VERBINDEN ZWEIER SCHAFTBAUTEILE**
CONNECTION APPARATUS FOR TWO SHAFT COMPONENTS, SURGICAL INSTRUMENT COMPRISING SUCH A CONNECTION APPARATUS, AND METHOD FOR CONNECTING TWO SHAFT COMPONENTS
APPAREIL DE RACCORDEMENT POUR DEUX COMPOSANTS D'ARBRE, INSTRUMENT CHIRURGICAL COMPRENANT UN TEL APPAREIL DE RACCORDEMENT, ET MÉTHODE DE RACCORDEMENT DE DEUX COMPOSANTS D'ARBRE

(30) Priorität: 30.06.2022 DE 102022116384
(43) Veröffentlichungstag der Anmeldung: 07.05.2025
(73) Patentinhaber: Karl Storz SE & Co. KG, 78532 Tuttlingen (DE)
(72) Erfinder: PÖNISCH, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); UNGER, Tobias, 78532 Tuttlingen (DE)
(86) Internationale Anmeldenummer: PCT/EP2023/067786
(87) Internationale Veröffentlichungsnummer: WO 2024/003225

(56) Entgegenhaltungen:
- DE-A1- 102011 121 989
- DE-A1- 4 238 619
- US-A- 5 308 358

## Beschreibung

Die Erfindung betrifft eine Verbindungsvorrichtung für zwei Schaftbauteile und ein chirurgisches Instrument, das eine solche Verbindungsvorrichtung zweier Schaftbauteile aufweist. Ferner betrifft die Erfindung ein Verfahren zum Verbinden zweier Schaftbauteile unter Verwendung der Verbindungsvorrichtung.

Aus dem Stand der Technik sind vielfältige chirurgische Instrumente bekannt, die es vorsehen, ein chirurgisches Werkzeug wie ein Endoskop, eine Zange oder ähnliches an einen Schaft anzudocken. Nach der Operation oder Behandlung des Patienten muss das chirurgische Instrument fachgerecht gereinigt werden, und hierfür müssen das von einem Werkzeugeinsatz mit Gabelschaft gehaltene Werkzeug und der Instrumentenschaft voneinander gelöst werden, die häufig mittels einer Bajonettverbindung gekoppelt sind. Dabei weist ein Schaftbauteil einen Nocken auf, der beim Einführen in das andere Schaftbauteil in einer Bajonettnut geführt wird, die sich vom Schaftende zunächst in axialer Richtung erstreckt und dann in Umfangsrichtung abknickt. Aus diesem Grund ist die Bajonettverbindung jedoch keine torsionssteife Verbindung und nur in gewissem Umfang biegesteif, dafür aber spielbehaftet.

Ein chirurgisches Instrument mit einer Bajonettverbindung zur lösbaren Verbindung zweier Rohrschaftinstrumente ist etwa aus DE 197 07 373 C1 bekannt.

Aus der Offenlegungsschrift DE 10 2011 121 989 A1 ist ein chirurgisches Rohrschaftinstrument mit einer lösbaren Verbindung mit Überwurfmutter bekannt.

DE 42 38 619 A1 lehrt ebenfalls ein mehrteiliges chirurgisches Instrument, dessen lösbare Verbindung mit einer Überwurfmutter gesichert wird.

Ausgehend von diesem Stand der Technik ist es Aufgabe der vorliegenden Erfindung, eine torsions- und biegesteife Verbindungsvorrichtung für zwei Schaftbauteile mit reduziertem Spiel bereitzustellen, die insbesondere zwei Schaftbauteile eines chirurgischen Instruments sein können.

Diese Aufgabe wird durch eine Verbindungsvorrichtung mit den Merkmalen des Anspruchs 1 gelöst.

Eine weitere Aufgabe besteht darin, ein chirurgisches Instrument mit einer verbesserten Verbindungsvorrichtung zweier Schaftbauteile bereitzustellen.

Diese Aufgabe wird durch das chirurgische Instrument mit den Merkmalen des Anspruchs 8 gelöst.

Die weitere Aufgabe, die Montage und Demontage zweier Schaftbauteile, die insbesondere Schaftbauteile eines chirurgischen Instruments sind, wird durch das Verfahren mit den Merkmalen des unabhängigen Anspruchs 9 gelöst.

Weiterbildungen der Verbindungsvorrichtung und des Verfahrens sind in den entsprechenden Unteransprüchen ausgeführt.

Gemäß einer ersten Ausführungsform einer erfindungsgemäßen Verbindungsvorrichtung für zwei Schaftbauteile mit einer gemeinsamen Längsachse weist ein erstes Schaftbauteil an einem verbindungsnahen Schaftende einen Aufnahmeraum auf, in dem ein Aufnahmeabschnitt an einem verbindungsnahen Schaftende eines zweiten Schaftbauteils aufgenommen werden kann. Dabei weist erfindungsgemäß das zweite Schaftbauteil an dem Aufnahmeabschnitt zumindest ein Formelement auf, das radial über einen Durchmesser des zweiten Schaftbauteils hinausragt, während das erste Schaftbauteil an dem Aufnahmeraum zumindest ein Aufnahmeelement aufweist, in dem das zumindest eine Formelement aufnehmbar ist. Hierdurch wird eine drehfeste Verbindung der beiden Schaftbauteile mittels Formschluss erreicht, die in axialer Richtung aber noch zusammen- bzw. auseinanderschiebbar sind.

Zur axialen Fixierung sieht das erste Schaftbauteil an dem verbindungsnahen Schaftende ein Außeneingriffselement vor, wobei die Verbindungsvorrichtung ein Mutterbauteil aufweist, das ein zum Eingriff mit dem Außeneingriffselement ausgebildetes Inneneingriffselement und einen Innenabsatz aufweist, der das Inneneingriffselement axialer Richtung bzw. die axiale Länge des Inneneingriffselements begrenzt. Das Mutterbauteil ist dabei drehbar auf dem zweiten Schaftbauteil auf einer von dem verbindungsnahen Schaftende abgewandten Seite des Formelements angeordnet und weist mit dem Inneneingriffselement in Richtung des verbindungsnahen Schaftendes und damit zu dem ersten Schaftelement. In einer Verbindungsanordnung der Verbindungsvorrichtung, in der das erste Schaftbauteil und das zweite Schaftbauteil verbunden sind, ist der Aufnahmeabschnitt mit dem Formelement des zweiten Schaftbauteils in dem Aufnahmeraum mit dem Aufnahmeelement des ersten Schaftbauteils aufgenommen, das Inneneingriffselement des Mutterbauteils steht mit dem Außeneingriffselement des ersten Schaftelements in Eingriff, und das in dem Aufnahmeelement aufgenommene Formelement liegt an dem Innenabsatz des Mutterbauteils an.

Damit stellt die erfindungsgemäße Verbindungsvorrichtung vorteilhaft eine spielfreie, torsions- und biegesteife Verbindung zweier Schaftbauteile bereit und ermöglicht darüber hinaus durch den Formschluss, dass die beiden Schaftbauteile ausgerichtet werden können.

Nach einer weiteren Ausführungsform der erfindungsgemäßen Verbindungsvorrichtung kann das Formelement ein Nocken sein, der radial überstehend an dem Aufnahmeabschnitt des zweiten Schaftbauteils ausgebildet ist. Das Aufnahmeelement kann dann einfach eine mit dem Aufnahmeraum verbundene Aufnahmenut sein, die sich in axialer Richtung von dem verbindungsnahen Schaftende erstreckt. In der Verbindungsanordnung ist der Nocken in der Aufnahmenut aufgenommen, wenn der Aufnahmeabschnitt des zweiten Schaftbauteils in den Aufnahmeraum des ersten Schaftbauteils durch den Eingriff des Innenkonturelements des Mutterbauteils mit dem Außenkonturelement des ersten Schaftelements eingeführt ist, und das Mutterbauteil mit dem Innenabsatz an dem Nocken anschlägt.

Nach einer dazu alternativen Ausführungsform der erfindungsgemäßen Verbindungsvorrichtung wird vorgeschlagen, dass das zumindest eine Formelement eine von einer Kreisform abweichende Querschnittsform des Aufnahmeabschnitts hat. Dabei weist das zumindest eine Aufnahmeelement mit dem Aufnahmeraum eine Querschnittsform auf, die mit der von der Kreisform abweichenden Querschnittsform des Aufnahmeabschnitts korrespondiert.

Gemäß einer noch weiteren Ausführungsform der erfindungsgemäßen Verbindungsvorrichtung kann das Außeneingriffselement am ersten Schaftbauteil vorteilhaft einfach ein Außengewinde und das Inneneingriffselement am Mutterbauteil ein mit dem Außengewinde korrespondierendes Innengewinde sein.

Nach einer dazu alternative Ausführungsform der erfindungsgemäßen Verbindungsvorrichtung wird vorgeschlagen, dass das Außeneingriffselement am ersten Schaftbauteil durch zumindest ein Nockenelement und das Inneneingriffselement durch zumindest eine hinterschnittene Bajonett-Nut oder umgekehrt das Außeneingriffselement durch zumindest eine hinterschnittene Bajonett-Nut und das Inneneingriffselement durch zumindest ein Nockenelement gebildet wird, wobei die hinterschnittene Bajonett-Nut einen zum verbindungsnahen Ende offenen Ausgangsabschnitt in axialer Richtung, einen Umfangsabschnitt und einen axialen Sackabschnitt aufweist, in dem das Nockenelement axial gefedert in der Verbindungsanordnung gehalten wird.

Nach einer noch weiteren Ausführungsform der erfindungsgemäßen Verbindungsvorrichtung wird vorgeschlagen, dass die Verbindungsvorrichtung eine Sicherungshülse aufweist, die auf dem zweiten Schaftbauteil auf einer von dem verbindungsnahen Schaftende abgewandten Seite des Mutterbauteils angeordnet und mit dem zweiten Schaftbauteil beispielsweise durch Gewinde, Kleben oder Schweißen verbunden ist. Die Sicherungshülse hält das Mutterbauteil verliersicher auf dem zweiten Schaftbauteil und stellt einen axialen Anschlag für das Mutterbauteil bereit.

Nach noch einer weiteren Ausführungsform der erfindungsgemäßen Verbindungsvorrichtung weist das erste Schaftbauteil einen Innenanschlag für den Aufnahmeabschnitt des zweiten Schaftbauteils auf. Der Innenanschlag kann in axialer Richtung fest begrenzt sein und durch einen in dem ersten Schaftbauteil ausgebildeten Innenabsatz bereitgestellt werden. Oder der Innenanschlag kann in axialer Richtung variabel durch einen Gewindestift einstellbar sein, der in ein Innengewinde, das angrenzend an den Aufnahmeraum in dem ersten Schaftbauteil ausgebildet ist, einschraubbar ist.

Schließlich kann bei einer erfindungsgemäßen Verbindungsvorrichtung nach einer weiteren Ausführungsform das Mutterbauteil an seiner Außenfläche gerändelt sein oder in axialer Richtung Einkerbungen aufweisen, um einen Kontakt mit den Fingern zum händischen Zuziehen oder Lösen der Verbindung zu verbessern.

Ein erfindungsgemäßes chirurgisches Instrument, das einen Instrumentenschaft aufweist, der an einem distalen Ende mit einem Gabelschaft eines Werkzeugeinsatzes verbunden ist, der ein Werkzeug hält, weist nach einer ersten Ausführungsform eine erfindungsgemäße Verbindungsvorrichtung auf, wobei der Instrumentenschaft das erste Schaftbauteil und der Gabelschaft das zweite Schaftbauteil bilden.

Ein erfindungsgemäßes Verfahren zum Verbinden zweier Schaftbauteile unter Verwendung einer erfindungsgemäßen Verbindungsvorrichtung umfasst nach einer ersten Ausführungsform die Schritte:
- drehbar anordnen des Mutterbauteils auf dem zweiten Schaftbauteil auf einer von dem verbindungsnahen Schaftende abgewandten Seite des zumindest einen Formelements und mit dem Inneneingriffselement in Richtung des verbindungsnahen Schaftendes weisend;
- ausrichten des ersten Schaftbauteils und des zweiten Schaftbauteils entlang der gemeinsamen Achse und bezüglich der radialen Position des zumindest einen Aufnahmeelements und des zumindest einen Formelements;
- zur Anlage bringen des Mutterbauteils mit dem Innenabsatz an dem Formelement;
- in Eingriff bringen des Inneneingriffselements des Mutterbauteils mit dem Außeneingriffselement des ersten Schaftelements, dabei einführen des zweiten Schaftbauteils mit dem Aufnahmeabschnitt in den Aufnahmeraum des ersten Schaftbauteils und aufnehmen des zumindest einen Formelements in dem zumindest eine Aufnahmeelement.

Nach einer weiteren Ausführungsform des erfindungsgemäßen Verfahrens wird vorgeschlagen, dass nach dem drehbar Anordnen des Mutterbauteils auf dem zweiten Schaftbauteil und vor dem Ausrichten der Schaftbauteile die Schritte erfolgen:
- anordnen einer Sicherungshülse als axialen Anschlag für das Mutterbauteil auf dem zweiten Schaftbauteil auf einer von dem verbindungsnahen Schaftende abgewandten Seite des Mutterbauteils; und
- verbinden der Sicherungshülse mit dem zweiten Schaftbauteil.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Dabei zeigen:
- Fig. 1: eine perspektivische Längsschnittansicht der Verbindungsvorrichtung nach einer erfindungsgemäßen Ausführungsform in Verbindungsanordnung,
- Fig. 2: eine Längsschnittansicht der erfindungsgemäßen Verbindungsvorrichtung in Verbindungsanordnung,
- Fig. 3: eine Detailansicht in einem um 90° gedrehten Längsschnitt des ersten Schaftbauteils und des Mutterbauteils der erfindungsgemäßen Verbindungsvorrichtung,
- Fig. 4: eine perspektivische Explosionsansicht der erfindungsgemäßen Verbindungsvorrichtung,
- Fig. 5: eine perspektivische Detailansicht der erfindungsgemäßen Verbindungsvorrichtung in Verbindungsanordnung,
- Fig. 6: eine perspektivische Ansicht der erfindungsgemäßen Verbindungsvorrichtung in getrennter Anordnung,
- Fig. 7: eine Seitenansicht eines chirurgischen Instruments mit einer erfindungsgemäßen Verbindungsvorrichtung in Verbindungsanordnung.

Die Erfindung bezieht sich auf eine Verbindungsvorrichtung zweier Schaftbauteile durch eine spielfreie, torsions- und biegesteife Verbindung mittels Formschluss, der zudem eine Ausrichtung der beiden Schaftbauteile erlaubt. Die Erfindung bezieht sich außerdem auf ein Verfahren zum Verbinden der zwei Schaftbauteile und auf ein chirurgisches Instrument mit Schaftbauteilen, die durch eine erfindungsgemäße Verbindungsvorrichtung gekoppelt sind.

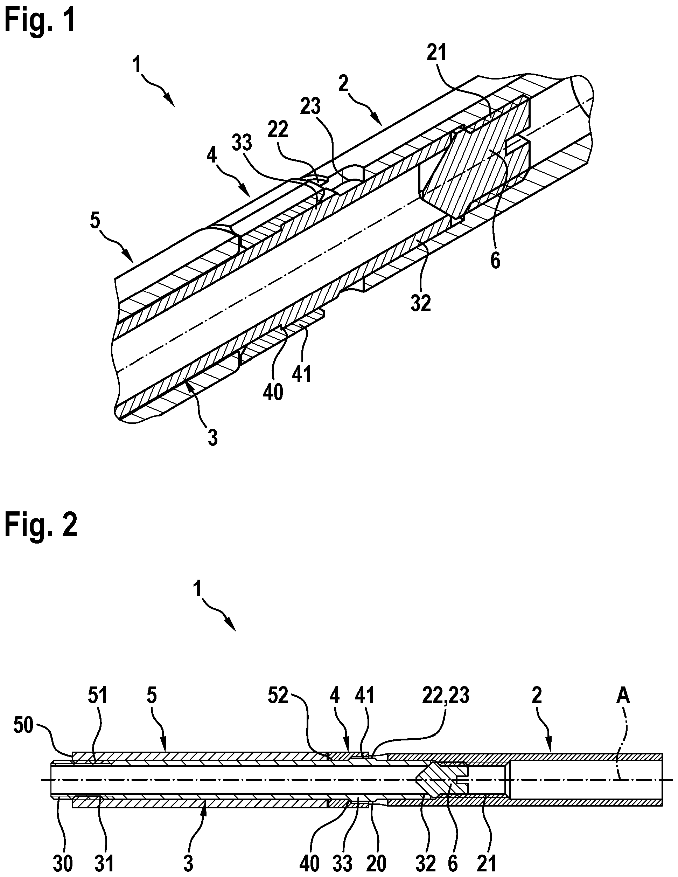

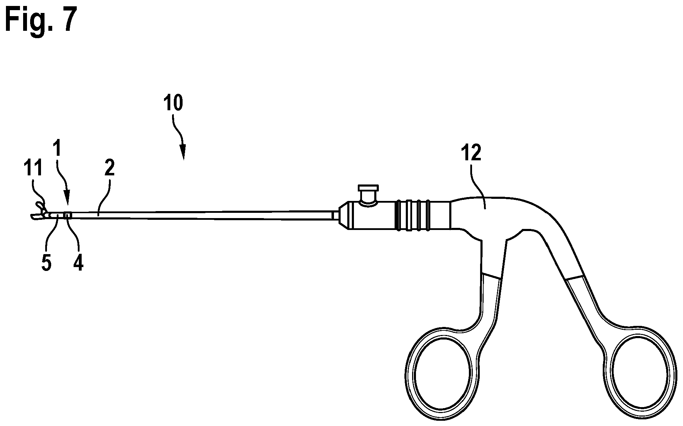

Zwar ist die Verbindungsvorrichtung 1 für zwei Schaftbauteile 2, 3, wie sie beispielhaft in Fig. 1 bis 6 dargestellt ist, insbesondere zum Einsatz in einem chirurgischen Instrument 10, wie in Fig. 7 dargestellt, vorgesehen und geeignet, ist allerdings nicht darauf beschränkt und kann auch auf anderen technischen Gebieten zur Verbindung zweier Schaftbauteile angewendet werden.

In einem chirurgischen Instrument 10 kann es sich bei den zu verbindenden Schaftbauteilen 2, 3 um einen Instrumentenschaft 2 und einen Gabelschaft 3 eines Werkzeugeinsatzes handeln, der zur Halterung des Werkzeugs 11 am distalen Ende des Instrumentenschafts 2 angeordnet und in Fig. 7 von einer Hülse 5 verdeckt ist. Nachfolgend werden daher das erste und das zweite Schaftbauteil 2, 3 in der Beschreibung der Verbindungsvorrichtung 1 im Zusammenhang mit Fig. 1 bis 6 auch als Instrumentenschaft 2 und Gabelschaft 3 bezeichnet, wobei die beschriebenen Ausführungen auch für andere zu verbindende Schaftbauteile gleichermaßen gelten sollen. Hierzu gehören auch zu verbindende Schaftbauteile eines chirurgischen Instruments, die nicht der Instrumentenschaft und der Gabelschaft sind, sondern z. B. der Instrumentenschaft und ein Schaftelement der Handhabe 12 oder andere Schaftelemente.

Außerdem ist ein chirurgisches Instrument 10, das eine Verbindungsvorrichtung 1 zur Verbindung zweier Schaftbauteile 2, 3 aufweist, nicht auf die beispielhafte Ausführung in Fig. 7 beschränkt, sondern kann sowohl in Bezug auf das distale Werkzeug 11 als auch in Bezug auf die Handhabe 12 am proximalen Ende des Instrumentenschafts 2 abweichen. Verschiedene Ausführungen von Werkzeugen und Handhaben, die zu einem chirurgischen Instrument 10 kombiniert werden können, sind dem Fachmann bekannt.

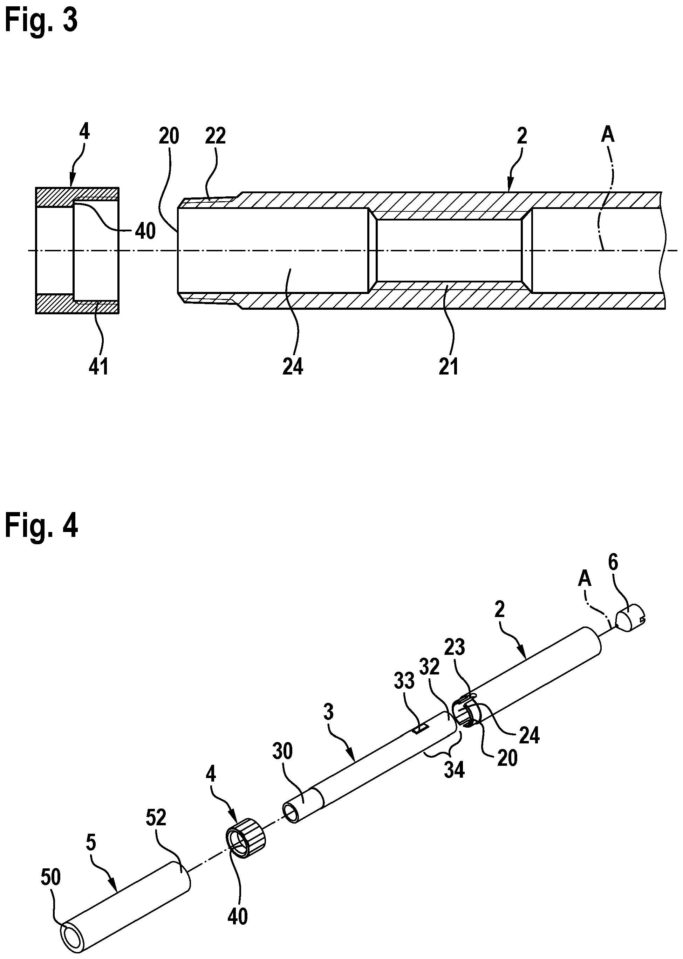

Fig. 1, 2 und 5 zeigen die erfindungsgemäße Verbindungsvorrichtung 1 in der Verbindungsanordnung der zwei Schaftbauteile 2, 3, die eine gemeinsame Längsachse A aufweisen. Das erste Schaftbauteil 2, beispielsweise der Instrumentenschaft 2, weist an einem verbindungsnahen Schaftende 20, d. h. dem Ende des Schaftbauteils 2, das dem zu verbindenden zweiten Schaftbauteil 3 zugewandt ist, einen Aufnahmeraum 24 auf, wie in Fig. 3, 4 und 6 zu sehen ist. Der Aufnahmeraum 24 ist zur Aufnahme eines Aufnahmeabschnitts 34 am verbindungsnahen Schaftende 32 des zweiten Schaftbauteils 3, beispielsweise des Gabelschafts 3, ausgebildet.

An dem verbindungsnahen Schaftende 20 weist der Instrumentenschaft 2 in dem gezeigten Beispiel zwei diametral angeordnete Aufnahmenuten 23 als Aufnahmeelemente auf, von denen in Fig. 2, 4 und 6, in denen beide Aufnahmenuten 23 dargestellt sind, nur eine bezeichnet ist. Jede Aufnahmenut 23 erstreckt sich geradlinig von dem verbindungsnahen Ende 20 in axialer Richtung, ohne abzuknicken. Der Gabelschaft 3 ist entsprechend an dem Aufnahmeabschnitt 34 mit zwei radial überstehenden, diametral angeordneten Nocken 33 als Formelementen ausgebildet, die in der Verbindungsanordnung in den Aufnahmenuten 23 des ersten Schaftbauteils 2 aufgenommen sind, sodass die beiden Schaftbauteile 2, 3 drehfest verbunden sind.

Ferner ist das erste Schaftbauteil 2 an dem verbindungsnahen Schaftende 20 mit einem Außengewinde 22 als einem Außeneingriffselement ausgebildet, das insbesondere in Fig. 3 zu sehen ist, in der die Aufnahmenuten 23 - anders als in Fig. 2 - nicht der Schnittebene liegen. Dieses Außengewinde 22 ist zum Eingriff mit einem Innengewinde 41 eines Mutterbauteils 4 vorgesehen, das drehbar auf dem zweiten Schaftbauteil 3 auf einer von dem verbindungsnahen Schaftende 32 abgewandten Seite der Nockens 33 angeordnet ist, und mit dem Innengewinde 41 zu dem Instrumentenschaft 2 weist. Anders als bei üblichen Muttern ist das Innengewinde 41 des Mutterbauteils 4 durch einen Innenabsatz 40 in axialer Richtung begrenzt, sodass in der Verbindungsanordnung bei Eingriff des Innengewindes 41 des Mutterbauteils 4 mit dem Außengewinde 22 des Instrumentenschafts 2 die in den Aufnahmenuten 23 aufgenommenen Nocken 33 an dem Innenabsatz 40 des Mutterbauteils 4 in axialer Richtung zur Anlage kommen.

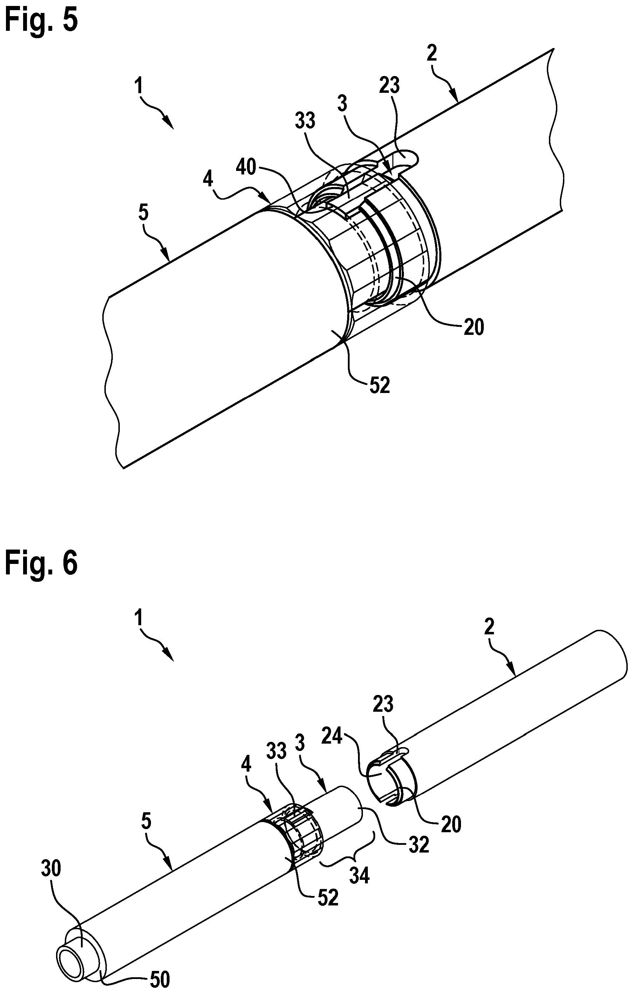

Das dargestellte Mutterbauteil 4 hat, wie insbesondere in Fig. 5 zu sehen, eine Außenkontur ähnlich einer Sterngriffmutter, damit die Verbindungsvorrichtung 1 werkzeuglos, d. h. händisch gefügt und getrennt werden kann. Die zylindrische Außenfläche des Mutterbauteils 4 weist dabei ringsum verteilte, axial verlaufende Einkerbungen auf, durch die sich im Querschnitt eine annähernd sternförmige Kontur ergibt, die sich gut mit den Fingern greifen und drehen lässt. Alternativ kann das Mutterbauteil beispielsweise ähnlich einer Rändelmutter einen zylindrischen Querschnitt mit gerändelter Außenfläche aufweisen, sodass das Mutterbauteil durch Fingerkontakt ohne Werkzeug einfach angezogen und gelöst werden kann. Selbstverständlich kann das Mutterbauteil aber auch eine andere Außenkontur aufweisen und beispielsweise entsprechend einer Mehrkantmutter, z. B. Sechskantmutter ausgebildet sein, die auch mit Fingerkraft oder ggf. einem Werkzeug gedreht werden kann. Die Gewindesteigung des Innengewindes 41 und des dazu korrespondierenden Außengewindes 22 können angepasst werden, so dass das Mutterbauteil beispielsweise nur durch eine geringe Drehung arretiert wird.

Zur Sicherung des Mutterbauteils 4 weist die Verbindungsvorrichtung 1 eine Sicherungshülse 5 auf, die als Gegenlager auf dem Gabelschaft 3 auf der Seite des Mutterbauteils 4 angeordnet ist, die von dem verbindungsnahen Schaftende 32 abgewandt ist. Die Sicherungshülse 5 dient gleichzeitig als axialer Anschlag und als Verliersicherung für das Mutterbauteil 4, das bei der Montage von dem verbindungsfernen Ende 30 des Gabelschafts 3 aufgeschoben werden muss, da es nicht über die Nocken 33 am Aufnahmeabschnitt 34 des Gabelschafts 3 geschoben werden kann. Der im Mutterbauteil 4 vorliegende radial abgesetzte Innenabsatz 40 schlägt in der Verbindungsanordnung an den Nocken 33 an und begrenzt die axiale Verschiebbarkeit des Mutterbauteils 4 über die Nocken 33 hinweg, wenn das drehbar um den Gabelschaft 3 angeordnete Mutterbauteil 4 auf den Instrumentenschaft 2 aufgeschraubt wird.

Die Sicherungshülse 5 wird mittels eines Innengewindes 51 am verbindungsfernen Ende 50 der Hülse 50 fest mit dem Gabelschaft 3 verbunden, der dazu ein entsprechendes Außengewinde 31 am verbindungsfernen Ende 30 aufweist. Alternativ zu einer solchen Schraubverbindung kann die Sicherungshülse 5 durch Kleben oder Schweißen oder eine andere Verbindungstechnik mit dem Gabelschaft 3 verbunden werden.

In der Verbindungsanordnung sichert das Mutterbauteil 4 die Verbindung axial, die Nocken 33 auf dem Gabelschaft 3 greifen in die Aufnahmenut 23 des Instrumentenschaftes 2 und sichern dies somit gegen Rotation. Dadurch wird das Mutterbauteil 4 nie auf Rotation belastet und kann sich nicht lösen oder fester zuziehen.

In der Verbindungsvorrichtung 1 weist der Instrumentenschaft 2 ferner einen Innenanschlag für das verbindungsnahe Ende 32 am Aufnahmeabschnitt 34 des Gabelschafts 3 auf. Der Innenanschlag wird durch einen Gewindestift 6 (hier eine Madenschraube, alternativ ist, z. B. auch eine Hohlschraube denkbar) bereitgestellt, der dargestellt ist. Der Gewindestift 6 ist mit seinem Außengewinde in ein Innengewinde 21 eingeschraubt, das angrenzend an den Aufnahmeraum 24 in dem Instrumentenschaft 2 ausgebildet ist. Die axiale Lage des Innenanschlags ist auf diese Weise durch die Einschraubtiefe des Gewindestifts 6 einstellbar.

Anders als dargestellt ist ein variabler Anschlag mittels Gewindestift nicht zwingend. Anstelle des Innengewindes für den Gewindestift 6 zur Einstellung des Innenanschlags für den Gabelschaft 3 kann der Instrumentenschaft 2 für einen feststehenden Anschlag einen Absatz aufweisen, der den Aufnahmeraum 24 begrenzt. Die Lage des Innenanschlags wird so gewählt bzw. eingestellt, dass die Verbindungsvorrichtung 1 in der Verbindungsanordnung vorliegt, wenn der Gabelschaft 3 mit dem verbindungsnahen Ende 32 an dem Innenanschlag anschlägt. Da beim Aufschrauben des Mutterbauteils 4 auf den Instrumentenschaft 2 der Innenabsatz 40 in dem Mutterbauteil 4 auf die Nocken 33 drückt, wird der Aufnahmeabschnitt 34 des Gabelschafts 3 in den Aufnahmeraum 24 des Instrumentenschafts 2 (und die Nocken 33 in der jeweiligen Aufnahmenut 23) bewegt, bis der Gabelschaft 3 den - hier von der Madenschraube 6 bereitgestellten - Innenanschlag kontaktiert. Auf diese Weise wird die Verbindung verspannt.

Die Länge der Aufnahmenuten 23 wird daher auf die Lage des Innenanschlags abgestimmt, damit die Nocken 33 in der Verbindungsanordnung nicht am Ende der Aufnahmenut 23 anschlagen und beim Aufschrauben des Mutterbauteils 4 die Einführtiefe des Aufnahmeabschnitts 34 durch die Lage des Innenanschlags und nicht durch die Länge der Aufnahmenut 23 begrenzt wird. In der Verbindungsanordnung in Fig. 1, 2 und 5 erstreckt sich der Nocken 33 daher in etwa bis zur Hälfte der Aufnahmenut 23 in axialer Richtung. Allerdings stellt die dargestellte Lage des Nockens 33 in der Aufnahmenut 23 keine Beschränkung dar: Der Abstand des Nockens 33 vom Ende der Aufnahmenut 23, bzw. die Länge eines axialen Reservewegs der Aufnahmenut 23, kann durchaus von dem dargestellten Beispiel abweichen, wenn sichergestellt ist, dass zuerst das eingeführte Schaftbauteil 3 an dem Innenanschlag anschlägt, bevor der Nocken 33 das Ende der Aufnahmenut 23 erreicht. Lassen es die Toleranzen zu, können die Lage des Innenanschlags und die Länge der Aufnahmenut auch so bemessen sein, dass das eingeführte Schaftbauteil 3 an dem Innenanschlag anschlägt und gleichzeitig der Nocken 33 das Ende der Aufnahmenut 23 erreicht.

In Fig. 1 bis 6 ist lediglich ein Beispiel für eine erfindungsgemäße Verbindungsvorrichtung 1 zur Verbindung zweier Schaftbauteile 2, 3 dargestellt, ohne dass die Erfindung auf das dargestellte Beispiel in allen Details beschränkt sein soll. Offensichtliche und naheliegende Variationen oder Abweichungen von dem dargestellten Beispiel sollen vom Schutzumfang als umfasst angesehen werden.

Dies betrifft beispielsweise die Anordnung und Anzahl der Nocken 33 am Gabelschaft 3 und die Anordnung und Anzahl der Aufnahmenuten 23 am Instrumentenschaft 2. Anders als im dargestellten Beispiel kann eine erfindungsgemäße Verbindungsvorrichtung auch nur einen Nocken und eine Aufnahmenut oder mehr als zwei Nocken und zwei Aufnahmenuten aufweisen. Bei einem einzigen Nocken-Aufnahmenut-Paar sowie bei nichtrotationssymmetrischer Anordnung von Nocken und Aufnahmenuten ist eine Kopplung der Schaftbauteile nur in einer vorbestimmten Ausrichtung der Schaftbauteile möglich. Eine rotationssymmetrische Anordnung von zwei oder mehr Nocken-Aufnahmenut-Paaren gestattet die Kopplung der Schaftbauteile in zwei oder mehr Rotationslagen.

Grundsätzlich ist es auch möglich, dass die Anzahl der Aufnahmenuten die Anzahl der Nocken übersteigt, wenn keine Ausrichtung der Rotationslage der Schaftbauteile zueinander erforderlich ist.

Eine nicht dargestellte Ausführungsform der erfindungsgemäßen Verbindungsvorrichtung 1 bezieht sich darauf, dass der Aufnahmeabschnitt des zweiten Schaftbauteils nicht Nocken als Formelemente aufweist, sondern mit einer von einer Kreisform abweichende Querschnittsform ausgebildet ist, die an zumindest einer Umfangsstelle über den Durchmesser des zweiten Schaftbauteil hinausragt. Dazu kann der Aufnahmeabschnitt beispielsweise eine polygonale, z. B. vier- oder sechseckige, oder elliptische oder ovale Querschnittsform haben, die jeweils an den Ecken oder den Scheitelpunkten der Hauptachse über den Querschnitt des zweiten Schaftbauteils hinausragt. Der Aufnahmeraum im ersten Schaftbauteil ist dann nicht mit Aufnahmenuten verbunden, sondern weist eine Querschnittsform auf, die mit der von der Kreisform abweichenden Querschnittsform des Aufnahmeabschnitts des zweiten Schaftbauteils korrespondiert.

Eine weitere nicht dargestellte Ausführungsform der erfindungsgemäßen Verbindungsvorrichtung 1 bezieht sich darauf, dass die Schraubverbindung zwischen Mutterbauteil 4 und erstem Schaftbauteil durch eine Bajonett-Verbindung mit Hinterschnitt ersetzt werden kann, welche axial gefedert in Position gehalten wird. Dabei wird das Außeneingriffselement am ersten Schaftbauteil durch zumindest ein Nockenelement und das Inneneingriffselement am Mutterbauteil durch zumindest eine hinterschnittene Bajonett-Nut bereitgestellt, oder umgekehrt, das Außeneingriffselement am ersten Schaftbauteil wird durch zumindest eine hinterschnittene Bajonett-Nut gebildet und das Inneneingriffselement am Mutterbauteil durch zumindest ein Nockenelement. Die hinterschnittene Bajonett-Nut erstreckt sich ausgehend vom verbindungsnahen Ende durch einen offenen Axialabschnitt, knickt in einen Abschnitt in Umfangsrichtung ab und endet in einem axialen Sackabschnitt, in dem das Nockenelement durch ein Federelement in axialer Richtung in der Verbindungsanordnung gehalten wird. Zum Lösen muss das Nockenelement entgegen der Federkraft aus dem Sackabschnitt bewegt werden, um durch den Umfangsabschnitt in den offenen Axialabschnitt zu gelangen, sodass das Mutterbauteil - mit dem zweiten Schaftbauteil - von dem ersten Schaftbauteil getrennt werden kann.

Das Verfahren zum Verbinden zweier Schaftbauteile 2, 3 umfasst, dass das Mutterbauteil 4 drehbar auf dem zweiten Schaftbauteil 3 auf einer von dem verbindungsnahen Schaftende 32 abgewandten Seite des Formelements (z. B. Nocken 33) so angeordnet wird, dass das Inneneingriffselement 41 in Richtung des verbindungsnahen Schaftendes 32 weist. Dazu wird das Mutterbauteil 4 von dem verbindungsfernen Ende 30 auf das zweite Schaftbauteil 3 aufgeschoben. Zur Sicherung des Mutterbauteils 4 kann anschließend eine Sicherungshülse 5 auf dem zweiten Schaftbauteil 3 auf einer von dem verbindungsnahen Schaftende 32 abgewandten Seite des Mutterbauteils 4 angeordnet und mit dem zweiten Schaftbauteil 3 verbunden werden. Das erste Schaftbauteil 2 und das zweite Schaftbauteil 3 werden bezüglich der radialen Position des Formelements/ Nockens 33 und des Aufnahmeelements/ der Aufnahmenut 23 ausgerichtet. Das Mutterbauteil 4 wird mit dem Innenabsatz 40 an dem Formelement / Nocken 33 zur Anlage gebracht. Es folgt das in Eingriff Bringen des Inneneingriffselements 41 des Mutterbauteils 4 mit dem Außeneingriffselement 22 des ersten Schaftelements 2, wobei das zweite Schaftbauteil 3 mit dem Aufnahmeabschnitt 34 in den Aufnahmeraum 24 des ersten Schaftbauteils 2 unter Aufnahme des Formelements bzw. Nockens 33 in dem Aufnahmeelement bzw. die Aufnahmenut 23 eingeführt wird, bis das zweite Schaftbauteil an einem feststehenden oder passend eingestellten Innenanschlag im ersten Schaftbauteil 2 anschlägt.

Die vorliegende Erfindung stellt eine Verbindungsvorrichtung 1 für zwei Schaftbauteile 2, 3 bereit, wobei ein erstes Schaftbauteil 2 einen Aufnahmeraum 24 aufweist, in dem ein Aufnahmeabschnitt 34 eines zweiten Schaftbauteils 3 aufnehmbar ist, das an dem Aufnahmeabschnitt 34 zumindest ein Formelement 33 aufweist, das radial über einen Durchmesser des zweiten Schaftbauteils 3 hinausragt. Das erste Schaftbauteil 2 weist an dem Aufnahmeraum 24 zur Aufnahme des Formelements 33 zumindest ein Aufnahmeelement 23 und an dem verbindungsnahen Schaftende 20 ein Außeneingriffselement 22 auf. Die Verbindungsvorrichtung 1 weist ein drehbar auf dem zweiten Schaftbauteil 3 angeordnetes Mutterbauteil 4 mit einem Inneneingriffselement 41 zum Eingriff mit dem Außeneingriffselement auf. Ein Innenabsatz 40 des Mutterbauteils 4 begrenzt das Inneneingriffselement 41 in axialer Richtung, sodass in einer Verbindungsanordnung des ersten Schaftbauteils 2 mit dem zweiten Schaftbauteil 3 der Aufnahmeabschnitt 34 mit dem Formelement 33 des zweiten Schaftbauteils 3 in dem Aufnahmeraum 24 und dem Aufnahmeelement 23 des ersten Schaftbauteils 2 aufgenommen ist, das Inneneingriffselement 41 des Mutterbauteils 4 mit dem Außeneingriffselement 22 des ersten Schaftelements 2 in Eingriff steht, und das in dem Aufnahmeelement 23 aufgenommene Formelement 33 an dem Innenabsatz 40 des Mutterbauteils 4 anliegt. Ferner werden ein chirurgisches Instrument 10 und ein Verfahren zum Verbinden zweier Schaftbauteile 2, 3 offenbart. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

### BEZUGSZEICHENLISTE

- 1: Verbindungsvorrichtung
- 10: Chirurgisches Instrument
- 11: Werkzeug
- 12: Handhabe
- 2: Erstes Schaftbauteil/Instrumentenschaft
- 20: Verbindungsnahes (distales) Instrumentenschaftende
- 21: Innengewinde
- 22: Außengewinde
- 23: Axialnut
- 24: Aufnahmeraum
- 3: Zweites Schaftbauteil/Gabelschaft
- 30: Verbindungsfernes (distales) Gabelschaftende
- 31: Außengewinde
- 32: Verbindungsnahes (proximales) Gabelschaftende
- 33: Nocken
- 34: Aufnahmeabschnitt
- 4: Mutterbauteil
- 40: Absatz
- 41: Innengewinde
- 5: (Sicherungs-)Hülse
- 50: Verbindungsfernes (distales) Hülsenende
- 51: Innengewinde
- 52: Verbindungsnahes (proximales) Hülsenende
- 6: Madenschraube (als einstellbarer Anschlag)
- A: Längsachse

## Patentansprüche

1. Verbindungsvorrichtung (1) für zwei Schaftbauteile (2, 3) mit einer gemeinsamen Längsachse (A), wobei ein erstes Schaftbauteil (2) an einem verbindungsnahen Schaftende (20) einen Aufnahmeraum (24) aufweist, in dem ein Aufnahmeabschnitt (34) an einem verbindungsnahen Schaftende (32) eines zweiten Schaftbauteils (3) aufnehmbar ist,
**dadurch gekennzeichnet, dass**
das zweite Schaftbauteil (3) an dem Aufnahmeabschnitt (34) zumindest ein Formelement (33) aufweist, das radial über einen Durchmesser des zweiten Schaftbauteils (3) hinausragt, und das erste Schaftbauteil (2) an dem Aufnahmeraum (24) zumindest ein Aufnahmeelement (23) aufweist, in dem das zumindest eine Formelement (33) aufnehmbar ist,
wobei das erste Schaftbauteil (2) an dem verbindungsnahen Schaftende (20) ein Außeneingriffselement (22) aufweist, und
die Verbindungsvorrichtung (1) ein Mutterbauteil (4) aufweist, das ein zum Eingriff mit dem Außeneingriffselement (22) ausgebildetes Inneneingriffselement (41) und einen Innenabsatz (40) aufweist, der das Inneneingriffselement (41) in axialer Richtung begrenzt,
wobei das Mutterbauteil (4) drehbar auf dem zweiten Schaftbauteil (3) auf einer von dem verbindungsnahen Schaftende (32) abgewandten Seite des Formelements (33) angeordnet ist und mit dem Inneneingriffselement (41) in Richtung des verbindungsnahen Schaftendes (32) weist,
sodass in einer Verbindungsanordnung des ersten Schaftbauteils (2) mit dem zweiten Schaftbauteil (3) der Aufnahmeabschnitt (34) mit dem Formelement (33) des zweiten Schaftbauteils (3) in dem Aufnahmeraum (24) und dem Aufnahmeelement (23) des ersten Schaftbauteils (2) aufgenommen ist, das Inneneingriffselement (41) des Mutterbauteils (4) mit dem Außeneingriffselement (22) des ersten Schaftelements (2) in Eingriff steht, und das in dem Aufnahmeelement (23) aufgenommene Formelement (33) an dem Innenabsatz (40) des Mutterbauteils (4) anliegt und wobei
das erste Schaftbauteil (2) einen Innenanschlag für den Aufnahmeabschnitt (34) des zweiten Schaftbauteils (3) aufweist, wobei der Innenanschlag
- in axialer Richtung fest begrenzt durch einen Innenabsatz bereitgestellt wird, oder
- in axialer Richtung variabel durch einen Gewindestift (6) oder eine Hohlschraube einstellbar ist, der in ein Innengewinde (21), das angrenzend an den Aufnahmeraum (24) in dem ersten Schaftbauteil (2) ausgebildet ist, einschraubbar ist.

2. Verbindungsvorrichtung (1) nach Anspruch 1,
**dadurch gekennzeichnet, dass**
das Formelement ein Nocken (33) ist, der radial überstehend an dem Aufnahmeabschnitt (34) des zweiten Schaftbauteils (3) ausgebildet ist, und das Aufnahmeelement eine mit dem Aufnahmeraum (24) verbundene Aufnahmenut (23) ist, die sich in axialer Richtung von dem verbindungsnahen Schaftende (20) erstreckt.

3. Verbindungsvorrichtung (1) nach Anspruch 1,
**dadurch gekennzeichnet, dass**
das zumindest eine Formelement durch eine von einer Kreisform abweichende Querschnittsform des Aufnahmeabschnitts (34) bereitgestellt wird, wobei das zumindest eine Aufnahmeelement mit dem Aufnahmeraum (24) eine Querschnittsform aufweist, die mit der von der Kreisform abweichenden Querschnittsform des Aufnahmeabschnitts (34) korrespondiert.

4. Verbindungsvorrichtung (1) nach zumindest einem der Ansprüche 1 bis 3,
**dadurch gekennzeichnet, dass**
das Außeneingriffselement (22) ein Außengewinde (22) und das Inneneingriffselement (41) ein mit dem Außengewinde (22) korrespondierendes Innengewinde (41) ist.

5. Verbindungsvorrichtung (1) nach zumindest einem der Ansprüche 1 bis 3,
**dadurch gekennzeichnet, dass**
das Außeneingriffselement (22) durch zumindest ein Nockenelement und das Inneneingriffselement (41) durch zumindest eine hinterschnittene Bajonett-Nut oder das Außeneingriffselement (22) durch zumindest eine hinterschnittene Bajonett-Nut und das Inneneingriffselement (41) durch zumindest ein Nockenelement gebildet wird, wobei die hinterschnittene Bajonett-Nut einen axialen Ausgangsabschnitt, einen Umfangsabschnitt und einen axialen Sackabschnitt aufweist, in dem das Nockenelement axial gefedert in der Verbindungsanordnung gehalten wird.

6. Verbindungsvorrichtung (1) nach zumindest einem der Ansprüche 1 bis 5,
**dadurch gekennzeichnet, dass**
die Verbindungsvorrichtung (1) eine Sicherungshülse (5) aufweist, die auf dem zweiten Schaftbauteil (3) auf einer von dem verbindungsnahen Schaftende (32) abgewandten Seite des Mutterbauteils (4) angeordnet und mit dem zweiten Schaftbauteil (3) verbunden ist.

7. Verbindungsvorrichtung (1) nach zumindest einem der Ansprüche 1 bis 6,
**dadurch gekennzeichnet, dass**
das Mutterbauteil (4) an seiner Außenfläche gerändelt ist oder in axialer Richtung Einkerbungen aufweist.

8. Chirurgisches Instrument (10) mit einem Instrumentenschaft (2), der an einem distalen Ende mit einem Gabelschaft (3) eines Werkzeugeinsatzes verbunden ist, der ein Werkzeug (11) hält,
**dadurch gekennzeichnet, dass**
das chirurgische Instrument (10) eine Verbindungsvorrichtung (1) nach zumindest einem der Ansprüche 1 bis 7 aufweist, wobei der Instrumentenschaft (2) das erste Schaftbauteil (2) und der Gabelschaft (3) das zweite Schaftbauteil (3) bilden.

9. Verfahren zum Verbinden zweier Schaftbauteile (2, 3) unter Verwendung einer Verbindungsvorrichtung (1) nach zumindest einem der Ansprüche 1 bis 7, umfassend die Schritte:
- drehbar anordnen des Mutterbauteils (4) auf dem zweiten Schaftbauteil (3) auf einer von dem verbindungsnahen Schaftende (32) abgewandten Seite des zumindest einen Formelements (33) und mit dem Inneneingriffselement (41) in Richtung des verbindungsnahen Schaftendes (32) weisend;
- ausrichten des ersten Schaftbauteils (2) und des zweiten Schaftbauteils (3) bezüglich der radialen Position des zumindest einen Aufnahmeelements (23) und des zumindest einen Formelements (33);
- zur Anlage bringen des Mutterbauteils (4) mit dem Innenabsatz (40) an dem Formelement (33);
- in Eingriff bringen des Inneneingriffselements (41) des Mutterbauteils (4) mit dem Außeneingriffselement (22) des ersten Schaftelements (2), dabei einführen des zweiten Schaftbauteils (3) mit dem Aufnahmeabschnitt (34) in den Aufnahmeraum (24) des ersten Schaftbauteils (2) und aufnehmen des zumindest einen Formelements (33) in dem zumindest eine Aufnahmeelement (23).

10. Verfahren nach Anspruch 9, das nach dem drehbar Anordnen des Mutterbauteils (4) auf dem zweiten Schaftbauteil (3) und vor dem Ausrichten der Schaftbauteile (2, 3) die Schritte umfasst:
- anordnen einer Sicherungshülse (5) für das Mutterbauteil (4) auf dem zweiten Schaftbauteil (3) auf einer von dem verbindungsnahen Schaftende (32) abgewandten Seite des Mutterbauteils (4); und
- verbinden der Sicherungshülse (5) mit dem zweiten Schaftbauteil (3).

## Claims

1. A connecting device (1) for two shaft components (2, 3) with a common longitudinal axis (A),
wherein a first shaft component (2) has, at a shaft end (20), close to the connection, a receiving space (24) in which a receiving section (34) at a shaft end (32), close to the connection, of a second shaft component (3) can be received,
**characterised in that**
the second shaft component (3) has, on the receiving section (34), at least one shaped element (33) which projects radially beyond a diameter of the second shaft component (3), and the first shaft component (2) has, on the receiving space (24), at least one receiving element (23), in which the at least one shaped element (33) can be received,
wherein the first shaft component (2) has an outer engagement element (22) at the shaft end (20) close to the connection, and
the connecting device (1) has a nut component (4) which has an inner engagement element (41) designed to engage with the outer engagement element (22) and an inner shoulder (40) which delimits the inner engagement element (41) in the axial direction,
wherein the nut component (4) is arranged rotatably on the second shaft component (3) on a side of the shaped element (33) which faces away from the shaft end (32), close to the connection, and points with the inner engagement element (41) in the direction of the shaft end (32) close to the connection,
such that, in a connection arrangement of the first shaft component (2) with the second shaft component (3), the receiving section (34) with the shaped element (33) of the second shaft component (3) is received in the receiving space (24) and in the receiving element (23) of the first shaft component (2), the inner engagement element (41) of the nut component (4) engages with the outer engagement element (22) of the first shaft element (2), and the shaped element (33) received in the receiving element (23) rests against the inner shoulder (40) of the nut component (4), and wherein
the first shaft component (2) has an inner stop for the receiving section (34) of the second shaft component (3), wherein the inner stop
- is provided in the axial direction as fixedly delimited by an inner shoulder, or
- can be variably set in the axial direction by way of a threaded pin (6) or a hollow screw, which can be screwed into an internal thread (21) which is formed in the first shaft component (2) adjoining the receiving space (24).

2. The connecting device (1) according to claim 1,
**characterised in that**
the shaped element is a cam (33) which is formed so as to protrude radially on the receiving section (34) of the second shaft component (3), and the receiving element is a receiving groove (23) which is connected to the receiving space (24) and which extends in the axial direction from the shaft end (20) close to the connection.

3. The connecting device (1) according to claim 1,
**characterised in that** the
at least one shaped element is provided by a cross-sectional shape of the receiving section (34) that deviates from a circular shape, wherein the at least one receiving element with the receiving space (24) has a cross-sectional shape that corresponds to the cross-sectional shape of the receiving section (34), which deviates from the circular shape.

4. The connecting device (1) according to at least one of claims 1 to 3,
**characterised in that**
the outer engagement element (22) is an external thread (22) and thep inner engagement element (41) is an internal thread (41) corresponding to the external thread (22).

5. The connecting device (1) according to at least one of claims 1 to 3,
**characterised in that**
the outer engagement element (22) is formed by at least one cam element and the inner engagement element (41) is formed by at least one undercut bayonet groove, or the outer engagement element (22) is formed by at least one undercut bayonet groove and the inner engagement element (41) is formed by at least one cam element, wherein the undercut bayonet groove has an axial outlet section, a circumferential section and an axial blind section, in which the cam element is held axially spring-loaded in the connection arrangement.

6. The connecting device (1) according to at least one of claims 1 to 5,
**characterised in that**
the connecting device (1) has a securing sleeve (5) which is arranged on the second shaft component (3) on a side of the nut component (4) facing away from the shaft end (32), close to the connection, and which is connected to the second shaft component (3).

7. The connecting device (1) according to at least one of claims 1 to 6, **characterised in that** the nut component (4) is knurled on its outer surface or has notches in the axial direction.

8. A surgical instrument (10) with an instrument shaft (2) which is connected at a distal end to a fork shaft (3) of a tool insert which holds a tool (11),
**characterised in that**
the surgical instrument (10) has a connecting device (1) according to at least one of claims 1 to 7, wherein the instrument shaft (2) forms the first shaft component (2) and the fork shaft (3) forms the second shaft component (3).

9. A method for connecting two shaft components (2, 3) using a connecting device (1) according to at least one of claims 1 to 7, comprising the steps:
- arranging the nut component (4) rotatably on the second shaft component (3) on a side of the at least one shaped element (33) facing away from the shaft end (32), close to the connection, and with the inner engagement element (41) pointing in the direction of the shaft end (32) close to the connection;
- aligning the first shaft component (2) and the second shaft component (3) with respect to the radial position of the at least one receiving element (23) and the at least one shaped element (33);
- bringing the nut component (4) with the inner shoulder (40) into abutment with the shaped element (33);
- bringing the inner engagement element (41) of the nut component (4) into engagement with the outer engagement element (22) of the first shaft elements (2), thereby introducing the second shaft component (3) with the receiving section (34) into the receiving space (24) of the first shaft component (2) and receiving the at least one shaped element (33) in the at least one receiving element (23).

10. The method according to claim 9, which, after arranging the nut component (4) rotatably on the second shaft component (3) and before aligning the shaft components (2, 3), comprises the steps of:
- arranging a securing sleeve (5) for the nut component (4) on the second shaft component (3) on a side of the nut component (4) facing away from the shaft end (32) close to the connection; and
- connecting the securing sleeve (5) to the second shaft component (3).

## Revendications

1. Dispositif de liaison (1) pour deux composants d'arbre (2, 3) comportant un axe longitudinal commun (A), dans lequel un premier composant d'arbre (2) présente, sur une extrémité d'arbre (20) proche de la liaison, un espace de logement (24) dans lequel une section de logement (34) peut être logée sur une extrémité d'arbre (32) proche de la liaison d'un second composant d'arbre (3),
**caractérisé en ce que**
le second composant d'arbre (3) présente, au niveau de la section de logement (34), au moins un élément façonné (33) qui dépasse radialement d'un diamètre du second composant d'arbre (3), et le premier composant d'arbre (2) présente, au niveau de l'espace de logement (24), au moins un élément de logement (23) dans lequel l'au moins un élément façonné (33) peut être logé,
dans lequel le premier composant d'arbre (2) présente un élément de mise en prise externe (22) sur l'extrémité d'arbre (20) proche de la liaison, et
le dispositif de liaison (1) présente un composant écrou (4) qui présente un élément de mise en prise interne (41) conçu pour venir en prise avec l'élément de mise en prise externe (22) et un épaulement interne (40) qui limite l'élément de mise en prise interne (41) dans la direction axiale,
dans lequel le composant écrou (4) est agencé de manière à pouvoir tourner sur le second composant d'arbre (3) sur un côté de l'élément façonné (33) détourné de l'extrémité d'arbre (32) proche de la liaison et est orienté avec l'élément de mise en prise interne (41) en direction de l'extrémité d'arbre (32) proche de la liaison,
de sorte que, dans un agencement de liaison du premier composant d'arbre (2) avec le second composant d'arbre (3), la section de logement (34) est logée avec l'élément façonné (33) du second composant d'arbre (3) dans l'espace de logement (24) et l'élément de logement (23) du premier composant d'arbre (2), l'élément de mise en prise interne (41) du composant écrou (4) est en prise avec l'élément de mise en prise externe (22) du premier composant d'arbre (2), et l'élément façonné (33) logé dans l'élément de logement (23) repose contre l'épaulement interne (40) du composant écrou (4), et dans lequel
le premier composant d'arbre (2) présente une butée interne pour la section de logement (34) du second composant d'arbre (3), dans lequel la butée interne
- est mise à disposition limitée de manière fixe dans la direction axiale par un épaulement interne, ou
- est réglable de manière variable dans la direction axiale par une filetée (6) ou une vis creuse, qui peut être vissée dans un filetage interne (21) qui est réalisé dans le premier composant d'arbre (2) de manière adjacente à l'espace de logement (24).

2. Dispositif de liaison (1) selon la revendication 1,
**caractérisé en ce que**
l'élément façonné est une came (33) qui est réalisée en saillie radiale sur la section de logement (34) du second composant d'arbre (3), et l'élément de logement est une rainure de logement (23) reliée à l'espace de logement (24), qui s'étend dans la direction axiale depuis l'extrémité d'arbre (20) proche de la liaison.

3. Dispositif de liaison (1) selon la revendication 1,
**caractérisé en ce que**
l'au moins un élément façonné est mis à disposition par une forme de section transversale de la section de logement (34) qui diffère d'une forme circulaire, dans lequel l'au moins un élément de logement comportant l'espace de logement (24) présente une forme de section transversale qui correspond à la forme de section transversale de la section de logement (34) qui diffère de la forme circulaire.

4. Dispositif de liaison (1) selon au moins l'une des revendications 1 à 3,
**caractérisé en ce que**
l'élément de mise en prise externe (22) est un filetage externe (22) et l'élément de mise en prise interne (41) est un filetage interne (41) correspondant au filetage externe (22).

5. Dispositif de liaison (1) selon au moins l'une des revendications 1 à 3,
**caractérisé en ce que**
l'élément de mise en prise externe (22) est formé par au moins un élément à came et l'élément de mise en prise interne (41) est formé par au moins une rainure à baïonnette à contre-dépouille ou l'élément de mise en prise externe (22) est formé par au moins une rainure à baïonnette à contre-dépouille et l'élément de mise en prise interne (41) est formé par au moins un élément à came, dans lequel la rainure à baïonnette à contre-dépouille présente une section de sortie axiale, une section périphérique et une section borgne axiale dans laquelle l'élément à came est maintenu axialement de manière élastique dans l'agencement de liaison.

6. Dispositif de liaison (1) selon au moins l'une des revendications 1 à 5,
**caractérisé en ce que**
le dispositif de liaison (1) présente une douille de sécurisation (5) qui est agencée sur le second composant d'arbre (3) sur un côté du composant écrou (4) détourné de l'extrémité d'arbre (32) proche de la liaison et qui est reliée au second composant d'arbre (3).

7. Dispositif de liaison (1) selon au moins l'une des revendications 1 à 6, **caractérisé en ce que** le composant écrou (4) est moleté sur sa surface extérieure ou présente des encoches dans la direction axiale.

8. Instrument chirurgical (10) comportant un arbre d'instrument (2) qui est relié à une extrémité distale à un arbre de fourche (3) d'un insert d'outil qui maintient un outil (11),
**caractérisé en ce que**
l'instrument chirurgical (10) présente un dispositif de liaison (1) selon au moins l'une des revendications 1 à 7, dans lequel l'arbre d'instrument (2) forme le premier composant d'arbre (2) et l'arbre de fourche (3) forme le second composant d'arbre (3).

9. Procédé de liaison de deux composants d'arbre (2, 3) à l'aide d'un dispositif de liaison (1) selon au moins l'une des revendications 1 à 7, comprenant les étapes :
- d'agencement de manière à ce qu'il puisse tourner du composant écrou (4) sur le second composant d'arbre (3) sur un côté de l'au moins un élément façonné (33) détourné de l'extrémité d'arbre (32) proche de la liaison et avec l'élément de mise en prise interne (41) orienté en direction de l'extrémité d'arbre (32) proche de la liaison ;
- d'alignement du premier composant d'arbre (2) et du second composant d'arbre (3) par rapport à la position radiale de l'au moins un élément de logement (23) et de l'au moins un élément façonné (33) ;
- de repos du composant écrou (4) avec l'épaulement interne (40) sur l'élément façonné (33) ;
- de mise en prise de l'élément de mise en prise interne (41) du composant écrou (4) avec l'élément de mise en prise externe (22) du premier composant d'arbre (2), insérant ainsi simultanément le second composant d'arbre (3) avec la section de logement (34) dans l'espace de logement (24) du premier composant d'arbre (2) et logeant l'au moins un élément façonné (33) dans l'au moins un élément de logement (23).

10. Procédé selon la revendication 9, qui, après l'agencement de manière à pouvoir tourner du composant écrou (4) sur le second composant d'arbre (3) et avant l'alignement des composants d'arbre (2, 3), comprend les étapes :
- d'agencement d'une douille de sécurisation (5) pour le composant écrou (4) sur le second composant d'arbre (3) sur un côté du composant écrou (4) détourné de l'extrémité d'arbre (32) proche de la liaison ; et
- liaison de la douille de sécurisation (5) avec le second composant d'arbre (3).
